# EUROPEAN PATENT APPLICATION

(11) **EP 4 492 045 A1**
(43) Date of publication of application: **15.01.2025**
(21) Application number: 23788014.1
(22) Date of filing: 15.02.2023
(51) Int. Cl.: G01N 21/67

(54) **EMISSION SPECTROSCOPIC ANALYSIS METHOD FOR SB IN METAL MATERIAL, METHOD FOR MEASURING SB CONCENTRATION IN MOLTEN STEEL DURING REFINING, AND METHOD FOR MANUFACTURING STEEL MATERIAL**

(30) Priority: 11.04.2022 JP 2022065064
(71) Applicant: JFE Steel Corporation, Tokyo 100-0011 (JP)
(72) Inventor: INOSE, Masao, Tokyo 100-0011 (JP); FURUYA, Ryosuke, Tokyo 100-0011 (JP)
(74) Representative: Hoffmann Eitle
(86) International application number: PCT/JP2023/005194
(87) International publication number: WO 2023/199591

(57) **Abstract**

Provided is an emission spectroscopic analysis method for measuring a concentration of Sb contained in a metal material (particularly, a metal material containing a large amount of iron, such as a steel material) in a speedy and highly accurate manner. The emission spectroscopic analysis method includes: repeatedly causing spark discharge between a metal material and an electrode facing the metal material to cause excitation emission; spectrally dispersing the excitation emission; and measuring an emission intensity I_{M} of a matrix element of the metal material and an emission intensity I_{Sb} of Sb contained in the metal material in order to obtain a ratio I_{Sb}/I_{M} of the emission intensity I_{Sb} to the emission intensity I_{M}, and the spark discharge is performed at a discharge energy of less than 0.10 J.

## Description

### TECHNICAL FIELD

The present invention relates to an emission spectroscopic analysis method for Sb in a metal material, a method for measuring an Sb concentration in molten steel during refining, and a method for manufacturing a semi-finished steel material (semi-finished product).

### BACKGROUND ART

Addition of a trace amount of a specific element into a steel material is effective for improvement of characteristics of the material and expression of a new function, and has been put to practical use.

For example, in the field of high tensile strength steel, Nb or Ti is added into a steel material at a level of several hundreds of ppm by mass. This is because Nb or Ti added to the steel material produces fine carbide, which brings about a precipitation strengthening action.

In the field of bearing steel, Sb is added to a steel material at a level of several tens of ppm by mass. This is because Sb added to the steel material suppresses a decrease in fatigue strength due to surface decarburization in a hot process such as hot forming or quenching.

Therefore, it is required to control the concentration of the element in the steel material without any excess or insufficiency so as to obtain targeted characteristics.

The concentration of the element in the steel material is adjusted during refining of the molten steel.

That is, a part of molten steel during refining is collected to prepare a sample, the concentration of a target element (referred to as "element A" for convenience) contained in the prepared sample is quickly analyzed, the amount of the element A to be added for bringing the concentration of the element A in the molten steel to a target concentration is calculated based on the analysis result, and the element A in the calculated amount is added to the molten steel.

Examples of the method used for quantification of an element in a steel material include a spark discharge atomic emission spectroscopic analysis method and a laser ablation ICP (inductively coupled plasma) mass spectrometry (LA-ICP-MS) .

In the spark discharge atomic emission spectroscopic analysis method, spark discharge is repeatedly caused between a metal material and an electrode, excitation emission occurred in each spark discharge is spectrally dispersed, and an emission intensity is measured.

Patent Literature 1 discloses a method for measuring a concentration of carbon contained in a metal material containing a large amount of iron, such as a steel material, in a speedy and highly accurate manner by using a spark discharge atomic emission spectroscopic analysis method.

### CITATION LIST

### PATENT LITERATURE

Patent Literature 1: JP 2014-215113 A

### SUMMARY OF INVENTION

### TECHNICAL PROBLEMS

However, when an Sb concentration in a steel material is measured by the method disclosed in Patent Literature 1, the sensitivity is low, and the Sb concentration at a level of several tens of ppm by mass cannot be measured.

Therefore, in order to accurately measure the Sb concentration in the steel material, it is necessary to separately perform wet chemical analysis. Since the wet chemical analysis requires a long time, the Sb concentration in molten steel during refining cannot be measured.

Therefore, the adjustment of the Sb concentration of molten steel is performed with reference to past results and predicted values based on refining parameters while the Sb concentration in molten steel is unknown.

As a result, the Sb concentration in the steel material as a final product may deviate from a target concentration, and in this case, the yield decreases.

The present invention has been made in view of the above points, and an object thereof is to provide an emission spectroscopic analysis method for measuring a concentration of Sb contained in a metal material (particularly, a metal material containing a large amount of iron, such as a steel material) in a speedy and highly accurate manner.

### SOLUTION TO PROBLEMS

The present inventors have conducted intensive studies, and as a result, have found that the above object is achieved by adopting the following configuration, thereby completing the present invention.

That is, the present invention provides the following [1] to [6].
[1] An emission spectroscopic analysis method for Sb in a metal material, the method comprising: repeatedly causing spark discharge between a metal material and an electrode facing the metal material to cause excitation emission; spectrally dispersing the excitation emission; and measuring an emission intensity I_{M} of a matrix element of the metal material and an emission intensity I_{Sb} of Sb contained in the metal material in order to obtain a ratio I_{Sb}/I_{M} of the emission intensity I_{Sb} to the emission intensity I_{M}, wherein the spark discharge is performed at a discharge energy of less than 0.10 J.
[2] The emission spectroscopic analysis method for Sb in a metal material according to [1], wherein the spark discharge is performed at a discharge energy of 0.07 J or less.
[3] The emission spectroscopic analysis method for Sb in a metal material according to [1] or [2], wherein the emission intensity is measured after a delay time.
[4] The emission spectroscopic analysis method for Sb in a metal material according to [3], wherein the delay time is longer than or equal to a time period in which the emission intensity of Sb for each spark discharge decreases to 60% of a maximum value.
[5] A method for measuring an Sb concentration in molten steel during refining, the method comprising: collecting a part of molten steel during refining to prepare an analysis sample; measuring an emission intensity I_{M} of a matrix element of the analysis sample and an emission intensity I_{Sb} of Sb contained in the analysis sample by using an emission spectroscopic analysis method; and obtaining an Sb concentration in the analysis sample based on a relationship between a ratio I_{Sb}/I_{M} obtained in advance and the Sb concentration using the ratio I_{Sb}/I_{M} of the emission intensity I_{Sb} to the emission intensity I_{M}, wherein the emission spectroscopic analysis method is the emission spectroscopic analysis method for Sb in a metal material according to any one of [1] to [4].
[6] A method for manufacturing a semi-finished steel material, the method comprising: obtaining an Sb concentration in molten steel during refining by using the method for measuring an Sb concentration in molten steel during refining according to [5]; determining an amount of Sb to be added to the molten steel during refining based on the obtained Sb concentration; and adding Sb to the molten steel during refining based on the determined amount of Sb to be added, whereby a semi-finished steel material is manufactured.

### ADVANTAGEOUS EFFECTS OF INVENTION

According to the present invention, the concentration of Sb contained in a metal material (particularly, a metal material containing a large amount of iron, such as a steel material) can be measured in a speedy and highly accurate manner.

### BRIEF DESCRIPTION OF DRAWINGS

[FIG. 1] FIG. 1 is a graph showing an example of a relationship between BEC and a discharge energy.
[FIG. 2] FIG. 2 is a graph showing an example of a relationship between BEC and a delay time.
[FIG. 3] FIG. 3 is a graph showing emission behavior of Fe and Sb for each spark discharge.
[FIG. 4] FIG. 4 is a graph showing a relationship between an emission intensity of Sb and BEC.

### DESCRIPTION OF EMBODIMENTS

### [Findings obtained by present inventors]

The present inventors have focused on a spark discharge atomic emission spectroscopic analysis method, and studied conditions for quantifying Sb at a level of several tens of ppm by mass in a steel material.

In order to increase the sensitivity of the spark discharge atomic emission spectroscopic analysis method, it is effective to lower the value of background equivalent concentration (BEC) that is considered to be correlated with the lower limit of quantitation.

The BEC is obtained by converting a signal intensity obtained from a sample free of element to be analyzed (the content of the element to be analyzed is zero) into a concentration, and is obtained from an absolute value (|b/a|) obtained by dividing an intercept "b" by slope "a" of a calibration curve (y = ax + b).

In the spark discharge atomic emission spectroscopic analysis method, in order to suppress variations derived from the sample, a ratio (I_{A}/I_{M}) of an emission intensity I_{A} of an element A to be analyzed to an emission intensity I_{M} of a matrix element is used as a signal intensity.

Thus, in order to lower the BEC, it is preferable to increase the slope "a", that is, to increase the value of the ratio (I_{A}/I_{M}).

Therefore, the present inventors have changed various analysis conditions, and obtained the BEC each time and compared the BEC.

As a result, the present inventors have found that a behavior peculiar to Sb appears in a relationship between an energy of spark discharge (discharge energy) and the BEC and a relationship between a delay time provided when measuring an excitation emission after each spark discharge and the BEC.

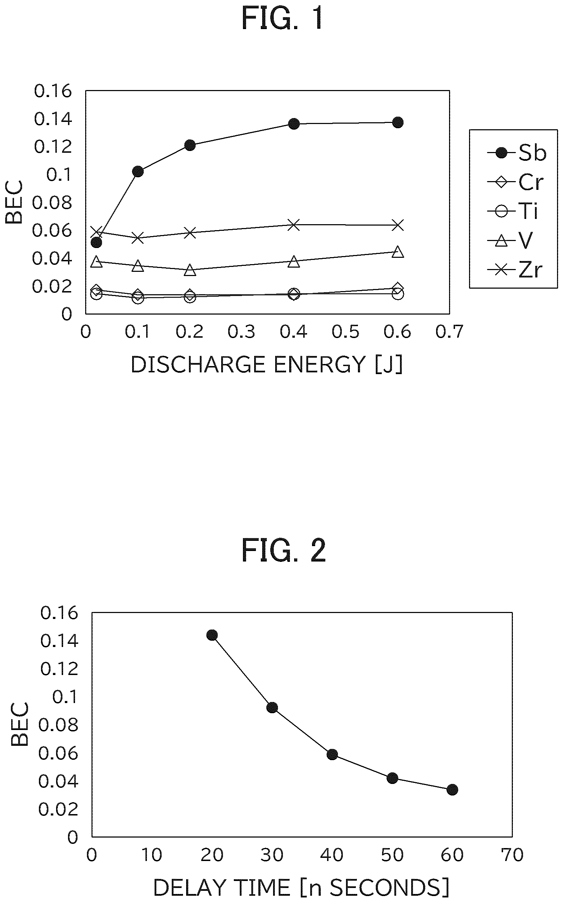

FIG. 1 is a graph showing an example of a relationship between BEC and a discharge energy.

As shown in FIG. 1, when elements to be analyzed are Cr, Ti, V, and Zr, the value of BEC hardly changed even when the discharge energy (unit: J) was changed. On the other hand, when an element to be analyzed was Sb, the value of BEC decreased by decreasing the discharge energy.

FIG. 2 is a graph showing an example of a relationship between BEC and a delay time.

As shown in FIG. 2, when an element to be analyzed is Sb, the BEC was reduced by providing a delay time (unit: n seconds).

The present invention has been made based on the above findings.

A preferred embodiment of the present invention will be described below.

### [Emission spectroscopic analysis method for Sb in metal material]

First, an emission spectroscopic analysis method for Sb in a metal material of the present embodiment (hereinafter, also referred to as "present analysis method") will be described.

### <Device used in emission spectroscopic analysis method for Sb in metal material>

A device used in the present analysis method is not particularly limited as long as it is a spark discharge atomic emission spectroscopic analysis device, but a solid-state emission spectroscopic analysis device of a type that simultaneously measures a plurality of elements is preferable.

### <Metal material>

The metal material as a sample of the present analysis method is not particularly limited, but the present analysis method is particularly effective for a metal material containing a large amount of iron, and thus a metal material containing a large amount of iron is preferable. That is, a matrix element of the metal material is preferably iron (Fe).

The metal material as a sample of the present analysis method may contain antimony (Sb) as an element to be analyzed.

As such a sample (metal material), for example, a sample collected from molten steel during refining or the like, the sample being solidified and then sheared, a shear surface of which is polished; a sample obtained by shearing a steel material and polishing a shear surface; and the like are used.

### <Spark discharge>

An electrode is disposed to face a metal material, and a spark discharge is caused plural times between the metal material and the electrode in an inert gas or in a vacuum to cause excitation emission. The spark discharge may be caused by a known method.

The number of times of spark discharge (the number of pulses) may be set to an appropriate number of times that can be analyzed with high accuracy. Preliminary discharge may be performed as necessary. The number of times of spark discharge for collecting data for analysis is preferably about 1000 to 2000 times.

Since the repetition frequency of the spark discharge does not particularly greatly contribute to the analysis performance, a general frequency of 200 to 400 Hz is preferable.

In the present embodiment, the energy of spark discharge (discharge energy) is less than 0.10 J. As a result, a value of a ratio (I_{Sb}/I_{M}) of an emission intensity I_{Sb} of Sb to the emission intensity I_{M} of the matrix element increases, and the BEC can be reduced.

The discharge energy is preferably 0.07 J or less and more preferably 0.05 J or less.

On the other hand, when the discharge energy is too low, the variation in the ratio (I_{Sb}/I_{M}) becomes large, and the accuracy may decrease. Therefore, the discharge energy is preferably 0.01 J or more and more preferably 0.02 J or more.

### <Spectral dispersing>

The excitation emission caused by performing the spark discharge is spectrally dispersed to obtain a specific spectral line for each element.

### <Measurement of emission intensity>

Subsequently, the emission intensity of specific spectral line for each element obtained by spectrally dispersing the excitation emission is measured. In this way, the emission intensities of Sb and the matrix element are measured.

In the measurement of the emission intensity I_{Sb} of Sb, an emission line is not particularly limited, but when the sample is a metal material containing a large amount of iron, it is preferable to use an emission line of 217.58 nm, which is a wavelength at which spectral interference is small.

An emission line for measuring the emission intensity I_{M} of the matrix element is not particularly limited as long as it is an emission line derived from the matrix element, has no overlap with other elements, and can obtain a sufficient intensity. For example, when the sample (metal material) is a steel material, examples of an emission line of Fe as a matrix element include Fe(I) 287.2 nm and Fe(II) 271.4 nm.

### <Delay time>

The present inventors have investigated the effect of delay time in capturing excitation emission for each spark discharge. The result showed that the BEC was lower in a case where the emission intensity was measured after a certain delay time from the spark discharge than in a case where the emission intensity was measured immediately after the spark discharge (FIG. 2).

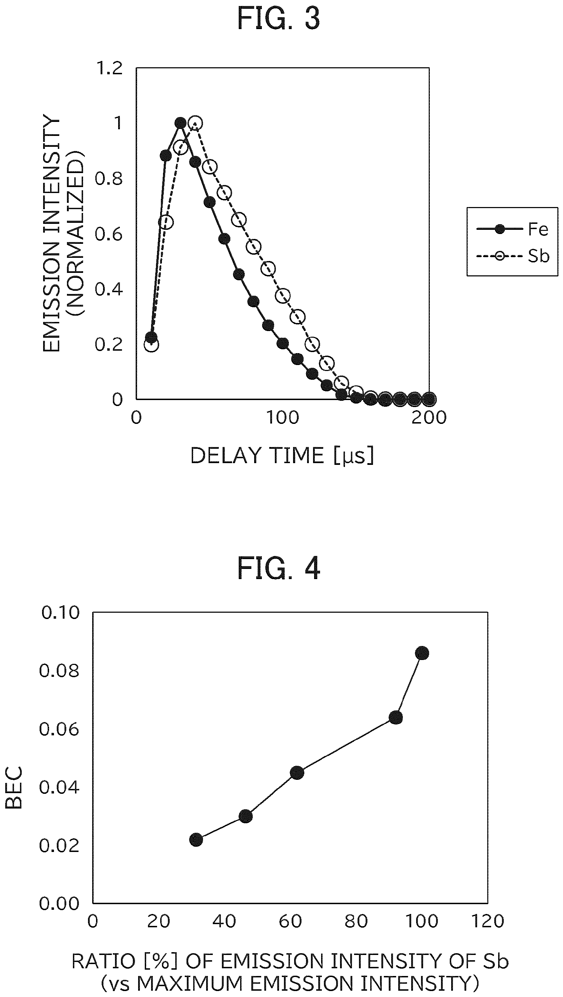

FIG. 3 is a graph showing emission behavior of Fe and Sb for each spark discharge, where the horizontal axis represents the elapsed time (unit: µs) from spark discharge, and the vertical axis represents the normalized emission intensity.

As for the vertical axis of FIG. 3, in order to compare elements having different absolute values of emission intensity, the emission intensity of each element is corrected so that the minimum value is zero and the maximum value is 1. That is, the emission intensity is normalized.

As shown in the graph of FIG. 3, Sb tends to emit light slightly later than iron (Fe) that is a matrix element. In view of the foregoing, it is considered that when the delay time is provided, the background is lowered due to the dimming of Fe, and at the same time, the light emission of Sb becomes relatively dominant, and the BEC is lowered.

FIG. 4 is a graph showing a relationship between an emission intensity of Sb and BEC.

In the case of a metal material containing a large amount of iron, the delay time may be set to a time period in which the emission intensity of Sb for each spark discharge decreases below the maximum value (maximum emission intensity).

Specifically, the delay time is preferably equal to or longer than a time period in which the emission intensity of Sb for each spark discharge decreases to 90% of the maximum value (maximum emission intensity), more preferably equal to or longer than a time period in which the emission intensity thereof decreases to 80% of the maximum value, and still more preferably equal to or longer than a time period in which the emission intensity thereof decreases to 60% of the maximum value.

On the other hand, when the delay time is too long, the BEC is lowered, but the emission intensity varies, and the accuracy in quantifying Sb may deteriorate.

Therefore, the delay time is preferably equal to or shorter than a time period in which the emission intensity of Sb for each spark discharge decreases to 20% of the maximum value (maximum emission intensity) and more preferably equal to or shorter than a time period in which the emission intensity thereof decreases to 30% of the maximum value.

A time (gate width) for capturing excitation emission after the lapse of the delay time is not particularly limited as long as the emission intensity sufficient for the quantification of Sb can be measured. The gate width of a certain time after the lapse of the delay time may be provided, or the gate width may be provided until the light is completely quenched after the lapse of the delay time.

### [Method for measuring Sb concentration in molten steel during refining]

Next, a method for measuring an Sb concentration in molten steel during refining of the present embodiment (hereinafter, also referred to as "present measurement method") will be described.

### <Preparation of analysis sample>

In the present measurement method, first, an analysis sample is prepared from molten steel during refining.

Specifically, a part of molten steel is collected from molten steel during refining, solidified, and then sheared to an appropriate size. Thereafter, a shear surface is polished to obtain an analysis sample.

### <Measurement of emission intensities of Sb and matrix element>

Subsequently, of the prepared analysis sample, the emission intensity I_{M} of the matrix element and the emission intensity I_{Sb} of Sb are measured by using an emission spectroscopic analysis method.

In the present measurement method, the above-described present analysis method is used as an emission spectroscopic analysis method.

### <Measurement of Sb concentration>

The ratio (I_{Sb}/I_{M}) is then obtained from the measured emission intensity I_{M} and emission intensity I_{Sb}, and an Sb concentration in the analysis sample is obtained based on a relationship between the ratio (I_{Sb}/I_{M}) obtained in advance and the Sb concentration by using the obtained ratio (I_{Sb}/I_{M}).

Specifically, for example, a metal material (steel material or the like) whose Sb concentration is known and including a concentration range of Sb to be analyzed is prepared in advance as a reference sample. At this time, two or more kinds of reference samples having different Sb concentrations are prepared.

Thereafter, the ratio (I_{Sb}/I_{M})] of the reference sample is obtained by using the above-described present analysis method as an emission spectroscopic analysis method. Note that measurement conditions using an emission spectroscopic analysis method for the reference sample and the analysis sample are the same.

A calibration curve is prepared from the obtained relationship between the ratio (I_{Sb}/I_{M}) and the Sb concentration.

Such a calibration curve is prepared in advance, and the Sb concentration is calculated by substituting the ratio (I_{Sb}/I_{M}) of the analysis sample into the calibration curve.

### [Method for manufacturing semi-finished steel material]

Next, a method for manufacturing a semi-finished steel material of the present embodiment (hereinafter, also referred to as "present manufacturing method") will be described.

In the present manufacturing method, first, an Sb concentration in molten steel during refining is obtained by using the above-described present measurement method. Subsequently, an amount of Sb added to the molten steel during refining is determined based on the obtained Sb concentration. That is, the amount of Sb added is determined so that the Sb concentration in the molten steel during refining becomes a target concentration.

Thereafter, Sb is added to the molten steel during refining based on the determined amount of Sb to be added. In this way, a semi-finished steel material is manufactured.

According to the present manufacturing method, since the amount of Sb to be added to the molten steel during refining can be controlled with high accuracy, a semi-finished steel material having stable characteristics can be manufactured with a high yield.

### EXAMPLES

Hereinafter, the present invention will be specifically described with reference to Examples. However, the present invention is not limited to Examples described below.

### <Inventive Examples 1 and 2 and Comparative Example 1>

Of analysis samples (12 samples) collected from molten steel during refining, an emission intensity I_{Fe} of Fe as a matrix element and an emission intensity I_{Sb} of Sb were measured by using a spark discharge atomic emission spectroscopic analysis method, a ratio (I_{Sb}/I_{Fe}) of the emission intensity I_{Sb} of Sb to the emission intensity I_{Fe} of Fe was obtained, and the Sb concentration (unit: ppm by mass) was calculated.

In Inventive Examples 1 and 2, as a spark discharge atomic emission spectroscopic analysis method, the above-described present analysis method was used.

Specifically, in Inventive Example 1, the spark discharge (integrated discharge) was performed at a discharge energy of 0.02 J. The delay time was set to a time period after which an emission intensity of 50% of the maximum emission intensity was obtained as the emission intensity of Sb.

In Inventive Example 2, the spark discharge (integrated discharge) was performed at a discharge energy of 0.05 J. The delay time was set to a time period after which an emission intensity of 40% of the maximum emission intensity was obtained as the emission intensity of Sb.

On the other hand, in Comparative Example 1, a conventional spark discharge atomic emission spectroscopic analysis method under standard measurement conditions was used as a spark discharge atomic emission spectroscopic analysis method.

In any example, a calibration curve of the ratio (I_{Sb}/I_{Fe}) of the emission intensity I_{Sb} of Sb to the emission intensity I_{Fe} of Fe and the Sb concentration was prepared in advance by using an iron/steel sample having a known Sb concentration as a reference sample, and the Sb concentration was calculated using the calibration curve.

As an emission line of Sb, an emission line of Sb(I) 217.58 nm was used.

As an emission line of Fe, an emission line of Fe(I) 287.2 nm was used.

As an analysis sample, a cylindrical casting sample collected from molten steel in the middle of refining was used.

The casting sample was sliced at the center to obtain a shear surface, and the shear surface was polished using a belt sander (particle size: #80). Of the obtained polished surface, the emission intensity I_{Sb} of Sb and the emission intensity I_{Fe} of Fe were measured.

A chip sample of about several grams was collected from the sample after measuring the emission intensity I_{Sb} of Sb, and a part thereof was acid-dissolved to obtain a chemical analysis value (unit: ppm by mass) of the Sb concentration.

The measurement conditions of the emission intensity I_{Fe} and the emission intensity I_{Sb} using the emission spectroscopic analysis method were described in Table 1 below.

The Sb concentration (unit: ppm by mass) calculated from the ratio (I_{Sb}/I_{Fe}) was described in Table 2 below. In Table 2 below, chemical analysis values were also described.

### [Table 1]

**Table 1**

| | | Inventive Example 1 | | Inventive Example 2 | | Comparative Example 1 | |
|---|---|---|---|---|---|---|---|
| | | Preliminary discharge | Integrated discharge | Preliminary discharge | Integrated discharge | Preliminary discharge | Integrated discharge |
| Discharge energy | [J] | 0.2 | 0.02 | 0.2 | 0.05 | 0.2 | 0.10 |
| Number of pulses | [times] | 1200 | 1000 | 1200 | 1000 | 1200 | 1000 |
| Delay time | [µs] | - | 60 | - | 80 | - | 0 |
| Gate width | [µs] | - | 150 | - | 150 | - | 300 |

**Table 2**

| Analysis sample No. | Sb concentration [ppm by mass] | | | |
|---|---|---|---|---|
| | Measurement result | | | Chemical analysis value |
| | Inventive Example 1 | Inventive Example 2 | Comparative Example 1 | |
| 1 | 10 | 16 | 24 | 11 |
| 2 | 10 | 13 | 21 | 10 |
| 3 | 11 | 13 | 26 | 12 |
| 4 | 21 | 20 | 21 | 21 |
| 5 | 18 | 16 | 21 | 18 |
| 6 | 22 | 21 | 25 | 21 |
| 7 | 47 | 47 | 53 | 48 |
| 8 | 37 | 39 | 42 | 40 |
| 9 | 36 | 34 | 40 | 36 |
| 10 | 42 | 41 | 41 | 41 |
| 11 | 46 | 45 | 41 | 44 |
| 12 | 47 | 48 | 49 | 47 |

### <Summary of evaluation results>

As shown in Table 2 above, the measurement result of the Sb concentration in Comparative Example 1 was greatly deviated from the chemical analysis value. Specifically, when σD was obtained as an index indicating the accuracy (inaccuracy) of the measurement result, σD of Comparative Example 1 was 5.1 ppm by mass.

In Comparative Example 1, the deviation was large particularly at an Sb concentration of 30 ppm by mass or less.

This is apparently because, under the measurement conditions of Comparative Example 1, the lower limit of quantitation was around 40 ppm by mass, and it was difficult to quantify Sb at a concentration less than 40 ppm by mass.

On the other hand, the quantification results of the Sb concentration in Inventive Examples 1 and 2 were extremely well matched with the chemical analysis values. Specifically, σD of Inventive Example 1 was 1.2 ppm by mass, and σD of Inventive Example 2 was 1.9 ppm by mass.

Since the lower limit of quantitation under the measurement conditions of Inventive Example 1 was about 8 ppm by mass and the lower limit of quantitation under the measurement conditions of Inventive Example 2 was about 24 ppm by mass, it is considered that more accurate measurement results were obtained.

Conventionally, Sb was quantified using wet chemical analysis, and in this case, it took about one day from collection of a sample until a quantification result was obtained, but in Inventive Examples 1 and 2, it took about 15 minutes.

Therefore, it was shown that a trace amount of Sb contained in the steel material can be quantified in a speedy and highly accurate manner in Inventive Examples 1 and 2.

## Claims

1. An emission spectroscopic analysis method for Sb in a metal material, the method comprising: repeatedly causing spark discharge between a metal material and an electrode facing the metal material to cause excitation emission; spectrally dispersing the excitation emission; and measuring an emission intensity I_{M} of a matrix element of the metal material and an emission intensity I_{Sb} of Sb contained in the metal material in order to obtain a ratio I_{Sb}/I_{M} of the emission intensity I_{Sb} to the emission intensity I_{M},
wherein the spark discharge is performed at a discharge energy of less than 0.10 J.

2. The emission spectroscopic analysis method for Sb in a metal material according to claim 1, wherein the spark discharge is performed at a discharge energy of 0.07 J or less.

3. The emission spectroscopic analysis method for Sb in a metal material according to claim 1 or 2, wherein the emission intensity is measured after a delay time.

4. The emission spectroscopic analysis method for Sb in a metal material according to claim 3, wherein the delay time is longer than or equal to a time period in which the emission intensity of Sb for each spark discharge decreases to 60% of a maximum value.

5. A method for measuring an Sb concentration in molten steel during refining, the method comprising:
collecting a part of molten steel during refining to prepare an analysis sample;
measuring an emission intensity I_{M} of a matrix element of the analysis sample and an emission intensity I_{Sb} of Sb contained in the analysis sample by using an emission spectroscopic analysis method; and
obtaining an Sb concentration in the analysis sample based on a relationship between a ratio I_{Sb}/I_{M} obtained in advance and the Sb concentration using the ratio I_{Sb}/I_{M} of the emission intensity I_{Sb} to the emission intensity I_{M},
wherein the emission spectroscopic analysis method is the emission spectroscopic analysis method for Sb in a metal material according to any one of claims 1 to 4.

6. A method for manufacturing a semi-finished steel material, the method comprising:
obtaining an Sb concentration in molten steel during refining by using the method for measuring an Sb concentration in molten steel during refining according to claim 5;
determining an amount of Sb to be added to the molten steel during refining based on the obtained Sb concentration; and
adding Sb to the molten steel during refining based on the determined amount of Sb to be added, whereby a semi-finished steel material is manufactured.
